(19) Europäisches Patentamt
European Patent Office
Office européen des brevets

(11) Veröffentlichungsnummer: **0 374 267 A1**

(12) **EUROPÄISCHE PATENTANMELDUNG veröffentlicht nach Art. 158 Abs. 3 EPÜ**

(21) Anmeldenummer: **89907376.1**

(22) Anmeldetag: **30.03.89**

(86) Internationale Anmeldenummer:
**PCT/SU89/00084**

(87) Internationale Veröffentlichungsnummer:
**WO 89/11861 (14.12.89 89/29)**

(51) Int. Cl.[5]: **A61K 37/10, A61K 33/24, A61K 9/08, A61K 31/28**

(30) Priorität: **06.06.88 SU 4437541**

(43) Veröffentlichungstag der Anmeldung:
**27.06.90 Patentblatt 90/26**

(84) Benannte Vertragsstaaten:
**AT BE CH DE FR GB LI SE**

(71) Anmelder: **INSTITUT FIZICHESKOI KHIMII IMENI L.V.PISARZHEVSK OGO AKADEMII NAUK UKRAINSKOI SSR**
**pr. Nauki, 31**
**Kiev, 252028(SU)**

Anmelder: **KIEVSKY GOSUDARSTVENNY INSTITUT USOVERSHENSTVOVANIA VRACHEI**
**ul. Dororgozhitskaya, 9**
**Kiev, 252112(SU)**

(72) Erfinder: **VOLCHENSKOVA, Ilima Iliodorovna**
**ul. Teremkovskaya, 13-40**
**Kiev, 252207(SU)**
Erfinder: **MAIDANEVICH, Nadezhda Nikolaevna ul. Oktyabrskaya 13-49 Kievo-Svyatoshinsky raion**
**Kievskaya obl. Vishnevy, 255500(SU)**
Erfinder: **BUDARIN, Lev Ivanovich**
**ul. Semashko, 10-31**
**Kiev, 252142(SU)**
Erfinder: **TROKHIMENKO, Elena Petrovna**
**ul. Zelenaya, 13-45 Kievo-Svyatoshinsky raion**
**Kievskaya obl. Vishnevy 255500(SU)**
Erfinder: **KEISEVICH, Ljudvig Vladislavovich**
**ul. Geroev Sevastopolya, 17a-44**
**Kiev, 252124(SU)**
Erfinder: **SHALIMOV, Sergei Alexandrovich**
**ul. Bolshaya Zhitomirskaya, 8/4-2**
**Kiev, 252025(SU)**
Erfinder: **KOPYTIN, Sergei Nikolaevich**
**pr. Nauki, 35/2-36**
**Kiev, 252028(SU)**

(74) Vertreter: **von Füner, Alexander, Dr. et al**
**Patentanwälte v. Füner, Ebbinghaus, Finck**
**Mariahilfplatz 2 & 3**
**D-8000 München 90(DE)**

(54) **DERIVATE VON PLATIN MIT POLYANION DER DEOXYRIBONUKLEINSÄURE, VERFAHREN ZUR HERSTELLUNG UND PHARMAZEUTISCHES ANTI-TUMORMITTEL DARAUS.**

(57) Derivate des Platin(II) mit einem Polyanion der Desoxyribonukleinsäure haben allgemeine Formel:

EP 0 374 267 A1

$$\left\{\left[PtCl(NH_3)\right]_6(OH)_x(H_2O)_y\right\}_n\text{-}DNS^{-m}$$, worin

$DNS^{-m} = \left(C_{39}H_{55-r}O_{32}N_{15}P_4\right)_n^{-m}$, $n = 2000 \pm 200$;

bei $x = o$, $y = 12$, $m = 6n$, $r = 6$;

bei $x = 2$, $y = 10$, $m = 6n$, $r = 4$;

bei $x = 0$, $y = 6$, $m = 6n$, $r = 6$.

Verfahren zur Herstellung der genannten Verbindungen besteht darin, dass man die Umsetzung der Desoxyribonukleinsäure mit einer Molekularmasse nicht unter 8 $10^6$ D mit cis-Dichlorodiamminplatin(II) oder mit teilweise hydrolysiertem cis-Dichlorodiamminplatin(II) in einem wässerigen Medium oder in einer Pufferlösung mit einem pH-Wert von 7,0 bis 7,5 und einer Ionenstärke von 0,0l bis 0,02 bei einer Temperatur nicht unter dem Schmelzpunkt der Desoxyribonukleinsäure mit anschliessender Isolierung des Endproduktes durchführt.

Die beanspruchten Verbindungen weisen antineoplastische Aktivität auf. Das Arzneimittel mit antineoplastischer Wirkung enthält als Wirkstoff die erfindungsgemässe Verbindung, Poly {hexakis [chloroamminaquaplatin(II)]}-$\mu$ -desoxyribonukleat.

SUAA-38802.3

# DERIVATE DES PLATIN(II) MIT EINEM POLYANION DER DESOXYRIBONUKLEINSÄURE, VERFAHREN FÜR IHRE HERSTELLUNG UND ARZNEIMITTEL MIT ANTINEOPLASTISCHER WIRKUNG AUF IHRER GRUNDLAGE

## Gebiet der Technik

Die vorliegende Erfindung bezieht sich auf das Gebiet der bioanorganischen Chemie und insbesondere auf neue Stoffe, Derivate des Platin(II) mit einem Polyanion der Desoxyribonukleinsäure, auf ein Verfahren für ihre Herstellung und auf ein Arzneimittel auf ihrer Grundlage mit antineoplastischer Wirkung.

## Vorhergehender Stand der Technik

Gegenwärtig wächst die Sterblichkeit der Bevölkerung von bösartigen Geschwülsten verschiedener Lokalisation, insbesondere bei Personen im jungen und mittleren Alter überall an. In diesem Zusammenhang wird der Suche und der Untersuchung von Arzneimitteln mit antineoplastischer Wirkung grosse Aufmerksamkeit geschenkt.

Unter den chemotherapeutischen Präparaten, die als antineoplastische Agenzien verwendet werden, ist ein besonders aktives und umfassend anwendbares in der medizinischen Praxis synthetisches Präparat anorganischer Natur, cis-Dichlorodiaminplatin(II) (CDDP, cisplatin)/ Hand book of cancer combination chemotherapy, I985, Bleomycin, Peplomycin, Cisplatin, Nippon Kauaku, I986, p. 6-7, 26-27, 46-47, 6I-63/.

Als antineoplastisches Präparat wird CDDP bei Mono- und Polychemotherapie des Karzinoms des Urogenitalsystems, bei Geschwülten in Lungen, im Kopf, am Hals und des Zentralnervensystems, bei Lymphomen und in der Onkogynäkologie verwendet. Die Effektivität der Behandlung einer Eierstockgeschwulst bei der Anwendung des CDDP-Präparates in Kombination mit Vinblastin, Bleomycin, Aktinomycin und Prednisolon erreicht I00%.

Das CDDP ist jedoch ein hochtoxisches Präparat, es ruft Schädigung der Nieren, Brechreiz, Erbrechen, die Unterdrückung der medullären Blutbildung, Gehörfunktionsstörungen, allergische Reaktionen, elektrolytische Störungen und neurologische Symptome hervor.

Eine besonders gefährliche Nebenwirkung bei der Therapie mit dem CDDP-Präparat ist seine Nephrotoxizität, die die therapeutische Dosis des Präparats einschränkt.

Die Verletzung der Nierenfunktion ist mit der Schädigung von Knäuelchen und Kanälchen verbunden und kommt in der Erhöhung des Harnstoffpegels, des Kreatinins und der Harnsäure bei biochemischen Analysen des Blutes zum Ausdruck.

Die Unterdrückung der medullären Blutbildung kann auf die Dosis und die Dauer der Behandlung zurückzuführen sein. Das CDDP-Präparat beschädigt alle drei Stränge der Blutbildung. Das Auftreten von Leukozytenmangel liegt in einem Bereich von 0 bis 52%, Anämien von 9 bis 40%. Es gibt Fälle der hämolytischen Anämie.

Infolge einer hohen Nephro- und Hämotoxizität ist es bei Behandlung mit dem CDDP-Präparat notwendig, die Kontrolle der biochemischen Kenndaten und des Blutbildes systematisch durchzuführen.

Gegenwärtig sind auch umfassend Präparate mit neoplatischer Wirkung bekannt, die als Wirkstoff organische Stoffe synthetischer oder natürlicher Herkunft enthalten (Alkylierungsagenzien, Antimetabolite, Antibiotika, Alkaloide, Fermente, Hormone und andere).

Diese Präparate treten in ihrem antineoplastischen Wirkungsgrad hinter dem CDDP-Präparat zurück. Sie weisen ausserdem eine Nebenwirkung auf Herzmuskel, auf Blutbildungssystem und auf Magen-Darm-Trakt auf. Alle bekannten Präparate mit antineoplastischer Wirkung unterdrücken in einem oder anderem Masse die immunologische Reaktivität des Organismus.

## Offenbarung der Erfindung

Die angemeldeten Verbindungen, das Verfahren für ihre Herstellung und das Arzneimittel auf ihrer Grundlage sind neu und wurden in der Literatur nicht beschrieben. Der Erfindung liegt die Aufgabe zugrunde, neue Verbindungen zu entwickeln, die eine hohe antineoplastische Aktivität in Verbindung mit einer immunologischen immunomodulierenden Aktivität und eine niedrige Toxizität aufweisen und die als Wirkstoffe eines Arzneimittels mit antineoplastischer Wirkung Anwendung finden, sowie ein Verfahren für ihre Herstellung zu entwickeln.

Die Aufgabe wurde dadurch gelöst, dass neue Verbindungen, Derivate des Platin(II) mit einem Polyanion der Desoxyribonukleinsäure allgemeiner Formel angemdldet werden:

$\{[PtCl(NH_3)]_6(OH)_x H_2O)_y\}_n - DNS^{-m}$, worin $DNS^{-m}$ = $(C_{39}H_{55-r}O_{32}N_{I5}P_4)_n$

$n = 2000 \pm 200$

bei $x = 0$, $y = I2$, $m = 6n$, $r = 6$;

bei $x = 2$, $y = I0$, $m = 4n$, $r = 4$;

bei $x = 0$, $y = 6$, $m = 6n$, $r = 6$.

Die angemeldeten Verbindungen weisen antineoplastische Aktivität sowie immunomodulierende Wirkung auf, sie stellen niedrigtoxische Verbindungen dar und finden Anwendung in der Medizin als Wirkstoff eines Arzneimittels mit antineoplastischer Wirkung. Das Arzneimittel mit antineoplastischer Wirkung, das einen Wirkstoff und ein pharmazeutisches Verdünnungsmittel enthält, erfindungsgemäss, weist als Wirkstoff Poly{hexakis [chloramminaquaplatin(II)]} - μ - desoxyribonukleat folgender Formel auf:

$\{[PtCl(NH_3)]_6(H_2O)_6\}_n - DNS^{-m}$, worin $DNS^{-m}$ = $(C_{39}H_{49}O_{32}N_{I5}P_4)_n^{-m}$ $n = 2I00 \pm I00$, $m = 6n$.

Das erfindungsgemässe Präparat weist eine hohe antineoplastische Aktivität in Verbindung mit immunomodulierender Wirkung auf und findet Anwendung bei Chemotherapie des Eierstockkrebses, Lungen-, Leber- und Gallenblasekarzinoms. Das erfindungsgemässe Präparat wird vorzugsweise in Form von Injektionslösungen und -suspensionen angewendet. Erfindungsgemäss weist das Arzneimittel in Form von Injektionslösungen vorzugsweise einen Wirkstoff in einer Menge von 0,I5 Masse% und als Verdünnungsmittel wässeriger Lösung aus eine 0,085 Masse% Natriumchlorid und 0,53

Masse% Natriumzitrat auf. Das Arzneipräparat in Form von Suspensionen für Injektionen weist vorzugsweise von I,5 bis 3,7 Masse% eines Wirkstoffes und als Verdünnungsmittel von 0,85 bis 2,I0 Masse% einer wässerigen Natriumchloridlösung und von 0,53 bis I,3 Masse% Natriumzitrat auf.

Erfindungsgemäss, werden die erfindungsgemässen Verbindungen durch Umsetzung der Desoxyribonukleinsäure mit einer Molekularmasse nicht unter $8 \cdot I0^6$ D mit cis-Dichlordiaminplatin(II) oder mit teilweise hydrolysiertem cis-Dichlordiamminplatin(II) in einem wässerigen Medium oder in einer Pufferlösung mit einem pH-Wert von 7,0 bis 7,5 und einer Ionenstärke von 0,0I bis 0,02 bei einer Temperatur nicht unter dem Schmelzpunkt der Desoxyribonukleinsäure und darauffolgende Isolierung des Endproduktes hergestellt.

Beste Ausführungsvariante der Erfindung

Die erfindungsgemässen Verbindungen stellen feindisperse Pulver mit gelber Farbe, ohne Geruch, stabil an der Luft dar, die innerhalb eines Jahres ohne Veränderung ihrer Eigenschaften gelagert werden können. Der Schmelzpunkt ist für sie nicht charakteristisch.

Die erfindungsgemässe Verbindung, Poly{hexakis-[chloroammindiaquaplatin(II)]}-μ-desoxyribonukleat folgender Formel:

$\{[PtCl(NH_3)]_6(H_2O)_{I2}\}-\mu-DNS^{-m}$ (Verbindung I), worin $n = I900 \pm 90$, $m = 6n$, $DNS^{-m} = (C_{39}H_{49}O_{32}N_{I5}P_4)_n^{-m}$, verliert bei Erhitzung innerhalb von I0 Stunden bei einer Temperatur von I80 bis 200°C alle Wassermoleküle. Der Stoff lässt sich schlecht in Wasser, Methanol, Äthanol auflösen, er ist nichthygroskopisch. Seine Löslichkeit in Wasser bei einer Temperatur von 20°C beträgt 0,002%. Eine Lösung von 0,0I5 g des beanspruchten Stoffes in I000 ml einer Zitrat-Salz-Lösung (0,0I5 Mol NaCl + 0,00I5 Mol Natriumzitrat) bei einer Temperatur von 20°C hat einen pH-Wert von 5,42 (pH- potentiometrisch).

UV-Spektrum einer 0,00I84%iger Lösung der erfindungs-

gemässen Verbindung I in Wasser in einem Bereich von 230 bis 300 nm weist einen Absorptionsmaximum bei 268,8± 0,2 nm (lgE = 4,000, berechnet auf I Mol Nukleotids) auf IR-Spektrum weist Absorptionsbanden bei 340 $cm^{-I}$ (Pt-Cl-Gruppe), I000-I300 $cm^{-I}$ ($PO_4$-Gruppe), I320 $cm^{-I}$ ($PtNH_3$-Gruppe), I500-I700 $cm^{-I}$ (C=C, C=N, C=O-Gruppen), 3I00-3700 $cm^{-I}$ (C-H, N-H, O-H-Gruppen) auf.

Reflexionsspektrum der festen Verbindung I hat Koeffizienten der Spektralreflexion $R_{248,3}(\%)$ = 4,60; $R_{333,3}(\%)$ = I2,3; $R_{400,6}(\%)$ = 22,4; $R_{5I4,4}(\%)$ = 60,I. Die mittelviskose Molekularmasse der erfindungsgemässen Verbindung I ist gleich $6,2 \cdot I0^6$ D. Die erfindungsgemässe Verbindung, Poly{bis[hydroxochloramminaquaplatin(II)] - tetrakis[chloroammindiaquaplatin(II)]} - μ - desoxyribonukleat folgender Formel:

$\{[PtCl(NH_3)]_6(OH)_2(H_2O)_{I0}\}_n-DNS^{-m}$ (Verbindung II),

worin $DNS^{-m} = \left(C_{39}H_{5I}O_{32}N_{I5}P_4\right)_n^{-m}$ , n = 2000±I00, m = 4n, verliert bei Erhitzung innerhalb von 8 Stunden bei einer Temperatur von I80 bis 200°C alle Wassermoleküle. Die Löslichkeit der Verbindung II in Wasser bei einer Temperatur von 20°C beträgt 0,0075%. Eine Lösung von 0,0I8 g dieses Stoffes in I000 ml einer Zitrat-Salz-Lösung bei einer Temperatur von 20°C hat einen pH-Wert von > 5,38.

UV-Spektrum einer 0,002%igen Lösung der Verbindung II in Wasser weist in einem Bereich von 230 bis 300 nm einen Absorptionsmaximum bei 265,7±0,2 nm (lg$\xi$ = 4,000, berechnet auf I Mol Nukleotid) auf. IR-Spektrum weist Absorptionsbanden bei 340 $cm^{-I}$ (Pt-Cl-Gruppe), I000-I300 $cm^{-I}$ ($PO_4$-Gruppe), I340 $cm^{-I}$ ($PtNH_3$-Gruppe), I500-I700 $cm^{-I}$ (C=C, C=N, C=O-Gruppe), 3I00-3700 $cm^{-I}$ (-H, N-H, O-H-Gruppen), 3295 $cm^{-I}$ (PtOH-Gruppe) auf.

Die mittelviskose Molekularmasse der erfindungsgemässe Verbindung II ist gleich $6,0 \cdot I0^6$ D.

Die erfindungsgemässe Verbindung, Poly{hexakis[chloramminaquaplatin(II)]} - μ - desoxyribonukleat folgender Formel:

$\{[PtCl(NH_3)]_6(H_2O)_6\}_n-DNS^{-m}$ (Verbindung III), worin

$DNS^{-m} = \left( C_{39}H_{49}O_{32}N_{15}P_4 \right)^{-m}$, n = 2100±100, m = 6n, verändert bei der Erhitzung innerhalb von 12 Stunden bei einer Temperatur von 180 bis 200°C ihre Masse nicht. Der Stoff lässt sich schlecht in Wasser, in Methanol und Äthanol auflösen. Seine Löslichkeit in Wasser bei einer Temperatur von 20°C beträgt 0,0005% und in einer Lösung, die 18 mMol/l NaCl und 1,8 mMol/l Natriumzitrat enthält, beträgt diese 0,005%. Eine Lösung aus 0,015 g dieses Stoffes in 1000 ml einer Zitrat-Salz-Lösung bei einer Temperatur von 20°C hat einen pH-Wert von 5,44.

UV-Spektrum einer 0,002%igen Lösung der Verbindung III in der Zitrat-Salz-Lösung in einem Bereich von 230 bis 300 nm weist einen Absorptionsmaximum bei 266,2±0,2 nm ($\lg \varepsilon$ = 4,000, berechnet auf 1 Mol Nukleotid) auf.

IR-Spektrum weist Absorptionsbanden bei 340 $cm^{-1}$ (PtCl-Gruppe), 1000-1300 $cm^{-1}$ ($PO_4$-Gruppe), 1340 $cm^{-1}$ ($PtNH_3$-Gruppe), 1500-1700 $cm^{-1}$ (C=C, C=N, C=O-Gruppen), 3000-3700 $cm^{-1}$ (CN-, NH-, OH-Gruppen) auf.

Reflexionsspektrum der festen Verbindung III zeichnet sich durch R 267,1(%) = 8,97, $R_{333,0}$(%) = 26,28 aus.

Die mittelviskose Molekularmasse der erfindungsgemässen Verbindung III ist gleich $6,2 \cdot 10^6$ D.

Die erfindungsgemässen Verbindung weisen antineoplastische Aktivität in Verbindung mit einer immunomudulierenden Wirkung auf. Die beanspruchten Verbindungen und das erfindungsgemässe Arzneipräparat mit antineoplastischer Wirkung wurden in Versuchen an Tieren und in Kliniken an Menschen untersucht. Untersucht wurde ihre antineoplastische Aktivität gegenüber der überimpften Shvetz-Erythromyelose. Die antineoplastische Aktivität jedes der angemeldeten Stoffe bewertete man nach den Ergebnissen der Versuche, die an weissen rassenlosen Ratten mit einem Gewicht von 150 bis 200 g (in Gruppen zu je 10 Tieren) angestellt wurden. Den Tieren wurden $2 \cdot 10^8 - 2 \cdot 10^{10}$ Zellen des überimpften Stammes der Shvetz-Erythromyelose subkutan in die Hüfte inokuliert. Am 4. bis 6. Tag nach der Inokulation der Tumorzellen, das heisst in der Periode der logarythmischen Phase des Wachstums eines Tumors,

begann man, den Tieren die erfindungsgemässen Verbindungen einzuführen. Die angemeldeten Verbindungen wurden intraperitoneal dreimal mit einem Zeitabstand von 24 Stunden in Form von wässerigen Lösungen beziehungsweise wässerigen Suspensionen eingeführt.

Die erfindungsgemässen Verbindungen wurden in folgenden Dosen eingeführt:

Verbindung I - I7,I mg/kg
Verbindung II - I7,I-27,3 mg/kg
Verbindung III - 27,3 mg/kg.

Antineoplastische Wirkung der erfindungsgemässen Verbindungen wurde im Vergleich zur Kontrolle (ohne Anwendung der Präparate) und im Vergleich zu den CDDP- und DNS-Präparaten untersucht. Die eingeführte Dosis von CDDP betrug 8,I mg/kg und die von DNS - I8,0 mg/kg.

Die antineoplastische Aktivität bewertete man nach folgenden biologischen Kenndaten: Grad der Hemmung des Wachstums eines Tumors, der Uberlebenskraft von Tieren am IO. Tag nach der Inokulation vom Tumorzellen sowie der Lebensdauer von Tieren.

Die Prüfergebnisse sind in der Tabelle I angeführt.

Tabelle I

| Stoffe | Grad der Hemmung des Wachstums eines Tumors,% | Uberlebenskraft von Tieren, % | Lebensdauer in Tagen |
|---|---|---|---|
| CDDP | 78 | 80 | II,5±0,5 |
| DNS | 0 | IOO | I8,5±0,5 |
| Erfindungsgemässe Verbindung I | 84 | IOO | 2I,5±0,5 |
| Erfindungsgemässe Verbindung II | 94 | IOO | II,5±0,5 |
| Erfindungsgemässe Verbindung III | 95 | IOO | 2I,0±I,0 |
| Kontrolle | 0 | IOO | I4,5±0,5 |

Wie aus der Tabelle I zu ersehen ist, treten die erfindungsgemässen Verbindungen nicht hinter dem CDDP-Präparat zurück beziehungsweise übertreffen dasselbe nach den Kenndaten der antineoplastischen Aktivität. Insbesondere zeichnet sich die Verbindung III aus, die effektiver als das CDDP das Wachstum eines Tumors unterdrückt, die Überlebenskraft von Tieren um 20% und die Lebensdauer derselben um IO Tage, das heisst um das Zweifache, vergrössern.

Untersucht wurde die akute Toxizität der beanspruchten Verbindungen.

Die akute Toxizität der angemeldeten Verbindungen im Vergleich zum CDDP untersuchte man an weissen rassenlosen Ratten mit einem Gewicht von I50 bis 200 g und an weissen nichtlinearen Mäusen mit einem Gewicht von $30 \pm 3$ g, die einer Quarantäne mindestens während 20 Tage ausgesetzt wurden. Die erfindungsgemässen Verbindungen I und II in Form von wässerigen Suspensionen und in Form von wässerigen Lösungen sowie die Verbindung III und das CDDP in Form von Zitrat-Salz-Suspensionen oder in Form von Zitrat-Salz-Lösungen (pH von 7,0 bis 7,5 und mit einer Ionenstärke von 0,0I bis 0,02) führte man dreimal intraperitoneal mit einem Zeitabstand von 2 Stunden in einem Volumen zu je 0,5 ml für Mäuse und zu je 2,0 ml für Ratten ein. Die Gesamtdosen für die erfindungsgemässen Verbindungen I, II und III betrugen 34,0; 68,0; 84,0; I02,0; und I36,0 mg/kg, für das CDDP - 8,0; I0,0; I4,0; I6,0 und I8,0 mg/kg. Die Anzahl der krepierten Tiere in jeder Gruppe wurde alle 24 bis 48 Stunden registriert. In jeder Gruppe waren je I2 Tiere (6 Männchen und 6 Weibchen), die Versuche wurden zweimal wiederholt. Die Ergebnisse der Versuche sind in der Tabelle 2 angeführt.

Tabelle 2

| Kenndaten der Toxizität | CDDP in Lösung | Erfindungsgemässe Verbindung I | | II | | III | |
|---|---|---|---|---|---|---|---|
| | | in Lösung | in Suspension | in Lösung | in Suspension | in Lösung | in Suspension |
| $LD_{50}$, mg/kg | II,3 | 5I,8 | 5I,8 | 66,0 | 66,0 | I02,0 | 82,5 |
| $LD_{I00}$, mg/kg | I8,3 | 84,0 | 84,0 | I00,0 | I00,2 | I36,0 | I20,0 |

Die Prüfergebnisse zeigen, dass die beanspruchten Stoffe weniger toxisch als CDDP sind. Am wenigstens toxisch davon ist die erfindungsgemässe Verbindung III. Die Toxizität der erfindungsgemässen Verbindung III verringert sich insbesondere dann, wenn man sie in einer Lösung verwendet, ohne das Reaktionsgemisch der lyophilen Trocknung und der anschliessenden Entfernung von Natriumsalzen auszusetzen.

Die krepierten Tiere wurden obduziert und man führte hystologische Untersuchungen der Nieren und des Darmes durch. Die Tiere, die die beanspruchten Stoffe in einer unter der toxischen Dosis liegenden Menge verabreicht bekamen, wurden in I4 Tagen getötet. Die inneren Organe derselben wurden der Autopsie ausgesetzt.

Den hystologischen Untersuchungen zufolge waren die Ursachen des Todes der Tiere, die die toxischen Dosen des CDDP-Präparates und der beanspruchten Stoffe verabreicht bekamen, unterschiedlich. Bei der Einführung von CDDP in toxischen Dosen wird eine ausgeprägte Dystrophie der inneren Organe, akute Pseudonephrose, massive Knäuelchennekrosen und Stromaödem beobachtet. Im Darm wird eine nicht stark ausgepräfte Epitheldysplasie nachgewiesen.

Bei der Einführung der beanspruchten Stoffe in toxischen Dosen werden in den Nieren umwesentliche umkehrbare Änderungen festgestellt: ein nicht stark ausgeprägtes Stromaödem, vereinzelte punktenartige Blutergüsse in den Knäuelchen, dystrophische Änderungen der Kanälchen. Dabei ist jedoch die Dysplasie und Desquamation des Darmepithels, die Schädigung seiner regenerierenden Funktion und die Reduzierung der Anzahl von Mitosen zu verzeichnen.

Bei der Einführung der beanspruchten Stoffe in den unter der toxischen Dosis liegenden Mengen sind die obengenannten Änderungen in den Nieren und im Darm umkehrbar und sie werden in I4 Tagen vollständig regeneriert.

Es wurde im die immunologische Aktivität der beanspruchten Stoffe untersucht. Die immunologische Aktivität ermittelte man nach den Kenndaten, die den Einfluss der beanspruchten Stoffe auf die Funktionen der Immunität-Systeme charakterisieren: zelluläre und homorale Immunität.

Der Einfluss auf die zelluläre Immunität bewertete man nach der Zeit der Abstossung eines Allotransplantats der Haut eines Tumorträgers.

Der Einfluss auf die homorale Immunität (Antitelogenese) bewertete man nach der Menge von antikörperbildenden Zellen in der Milz und nach dem Titer von Agglutininen und Hämolysinen im Blutserum.

Der Einfluss der beanspruchten Stoffe auf die zelluläre Immunität wurde an Männchenratten der Wistar-Linie mit einem Gewicht von I50 bis 200 g untersucht. Den Tieren wurde ein Hautlappen mit Abmessungen 2 x 3 $cm^2$ von den Ratten der August-Linie in die Rückengegend transplantiert. Dann wurden denselben Tieren subkutan $2 \cdot IO^8$ Zellen der Shvetz-Erythromyelose in die Hüfte inokuliert. Am 4. bis 6. Tag nach der Uberimpfung eines Tumors führte man den Tieren intraperitoneal dreimal in einem Zeitabstand von 24 Stunden die erfindungsgemässe Verbindung I in einer Dosis von I7,I mg/kg, die Verbindung II in einer Dosis von I7,I mg/kg und die Verbindung III in einer Dosis von 27,3 mg/kg ein. Die Verbindungen I und II führte man in Form einer wässerigen Lösung und die Verbindung III in einer Lösung von 0,0I5 Mol Natriumchlorid + 0,00I5 Mol Natriumzitrat ein. Zum Vergleich verwendete man die DNS- und CDDP-Präparate, die amn in einer Salzlösung nach dem gleichen Schema in Dosen von I2,0 und

8,I mg/kg einführte. Als Kontrolle dienten intakte Tiere. Die Zeit der Abstossung des Hautlappens fixierte man nach der Änderung seiner Temperatur, Farbe und Elastizität.

Die Prüfergebnisse sind in der Tabelle 3 angeführt.

Tabelle 3

Die Zeit der Abstossung (in Tagen) eines Hauptlappens bei intakten Tieren und bei Tumorträgern ohne Behandlung und bei Behandlung mit den beanspruchten Stoffen

| Intakte Tiere | Tumorträger ohne Behandlung | Die behandelten Tumorträger | | | | |
|---|---|---|---|---|---|---|
| | | CDDP | DNS | Mit beanspruchten Stoffen | | |
| | | | | I | II | III |
| 9,5±I,0 | I5,0±I,0 | I3,5±0,5 | I2,5±0,5 | 6,0±I,0 | 6,5±0,5 | 6,5±0,5 |

Im Vergleich zu den intakten Tieren vergrössert sich die Zeit der Abstossung des Hauptlappens bei den nichtbehandelten Tumorträgern um 5,5 Tage. Das zeugt von der Senkung der zellulären Immunität des Organismus unter Bedingungen der Entwicklung eines Tumors.

Die Einführung der Präparate CDDP und DNS beeinflusst praktisch nicht die zelluläre Immunität eines Tumorträgers (CDDP) oder beeinflusst in einem unwesentlichen Masse (DNS). Die Einführung der erfindungsgemässen Verbindungen verringert die Zeit der Abstossung eines Hauptlappens um 3 Tage im Vergleich zu den intakten Tieren und um 8,5 Tage im Vergleich zu den nichtbehandelten Tumorträgern.

Hierdurch stimuliert die Einführung der erfindungsgemässen Verbindungen in einem beträchtlichen Masse die zelluläre Immunität eines Tumorträgers, indem ihr Niveau oberhalb des Niveaus eines gesunden Tieren gebracht wird, was bei der Einführung von CDDP und DNS nicht gelingt.

Der Einfluss der beanspruchten Stoffe auf die humorale Immunität wurde auch im Vergleich zum CDDP und zur DNS untersucht. Dafür wurden Ratten, Tumorträger der Shvetz-Erythromyelose der Wistar-Linie eingesetzt, denen

man ein Tumor subkutan in die Hüfte überimpfte. Am 4. Tag nach der Uberimpfung des Tumors begann man ihre Behandlung nach dem gleichen Schema wie auch bei der Untersuchung der zellulären Immunität. Am 8. Tag nach der Uberimpfung des Tumors führte man die Immunisierung der Tiere durch, indem man ihnen intraperitoneal Hammelerythrozyte in einer Dosis von $2 \cdot IO^6$ Zellen einführt. In 4. Tagen nach der Immunisierung tötete man die Tiere mittels Dekapitation unter Anwendung von Äthernarkose und man ermittelte den Gehalt an antikörperbildenden Zellen in der Milz nach der Erne-Methode und die Agglutinine- und Hämolysine-Titer im periphärischen Blut mit bekanntnn Methoden.

Der Gehalt an antikörperbildenden Zellen ermittelte man, berechnet auf $IO^6$ Splenozyte und auf die Gesamtmasse der Milz. Dee Versuchergebnisse sind in der Tabelle 4 angeführt.

Tabelle 4

| Lfd. Nr. | Kenndaten der humoralen Immunität | Gruppen von Tieren | |
|---|---|---|---|
| | | intakte Tiere | Tumorträger ohne Behandlung |
| I | 2 | 3 | 4 |
| I. | Gehalt an antikörperbildenden Zellen je $IO^6$ Splenozyte | 2530±550 | 283,7±22,5 |
| 2. | Gehalt an antikörperbildenden Zellen, berechnet auf die Milzmasse | (I828±250)x x$IO^3$ | (250±I6,8) x x$IO^3$ |
| 3. | Agglutinine-Titer | I:5I2 | I:I60 |
| 4. | Hämolysine-Titer | I:4064 | I:2235 |

Fortsetzung der Tabelle 4

| Lfd. Nr. | Gruppen von Tieren | | | | |
|---|---|---|---|---|---|
| | Behandelte Tumorträger | | | | |
| | CDDP | DNS | mit den beanspruchten Stoffen | | |
| | | | I | II | III |
| I | 5 | 6 | 7 | 8 | 9 |
| I. | 20,37±2,03 | 292,5±30,7 | 68,4±9,38 | 73,2I±I4,2 | 69,2±I2,7 |
| 2. | (36,42±2,03)x $xIO^3$ | (302,7± 45m3) $xIO^3$ | (78,96± 3,72) $xIO^3$ | (84,2I± 3,3I) $xIO^3$ | (56,25± IO,0) $xIO^3$ |
| 3. | I:64 | I:I28 | I:I28 | I:I28 | I:I00 |
| 4. | I:I28 | I:2560 | I:256 | I:650 | I:5I2 |

Die beanspruchten Stoffe verringern die humorale Immunität eines Tumorträgers. Sie verringern die Anzahl der antikörperbildenden Zellen und senken die Agglutinin- und Hämolysin-Titer. Die Verringerung der humoralen Immunität unter Einwirkung der beanspruchten Stoffe erfolgt jedoch in einem geringeren Masse als unter Einfluss von CDDP. Das DNS-Präparat beeinflusst die humorale Immunität praktisch nicht.

Der Einfluss der beanspruchten Stoffe auf die Antitelogenese wurde auch an zwei gegensätzlich auf Hammelerythrozyte reagierenden Mäuse-Linien: CBA und $C_{57}Bl_6$ untersucht. Man verwendete Tiere mit einem Gewicht von 20±2 g, die einer Quarantäne innerhalb von mindestens 20 Tagen ausgesetzt wurden. Den intakten Tieren führte man einmal intraperitoneal die beanspruchten Stoffe in Dosen von I3,6 mg/kg, die DNS in einer Dosis von 6,0 mg/kg und das CDDP in einer Dosis von 8,I mg/kg ein. In einer Stunde wurden die Tiere immunisiert, indem man ihnen intraperitoneal $IO^8$ Zellen der Hammelerythrozyte einführt. Am 7., I4. und 30. Tag tötete man die Tiere und ermittelte man den Gehalt an Agglutininen im Blutserum in an sich bekannter Weise. Die Ergebnisse wurden in Form $lg_2$ der Antikörper-Titer ausgedrückt. Die Ergebnisse sind in der Tabelle 5 angeführt.

Tabelle 5

| Einzuführende Stoffe, Dosis | Beobachtungszeit | $lg_2$ Antikörper-Titer Linie der Mäuse CBA | Linie der Mäuse $C_{57}Bl_6$ |
|---|---|---|---|
| Kontrolle | 7 | 8,95±0,I5 | 7,25±0,2 |
| (Ohne Einführung | I4 | 9,42±0,23 | 7,72±0,2 |
| der Stoffe) | 30 | 9,20±0,20 | 6,24±0,60 |
| CDDP (8,I mg/kg) | 7 | 5,30±0,I5 | 3,80±0,24 |
| | I4 | 5,80±0,I9 | 4,04±0,I2 |
| | 30 | 5,I0±0,30 | 4,2I±0,37 |
| Erfindungsgemässe | 7 | 7,30±0,I2 | 6,40±0,I5 |
| Verbindung I | I4 | 7,80±0,3I | 6,82±0,38 |
| (I3,6 mg/kg) | 30 | 8,I±0,40 | 6,25±0,3I |
| Erfindungsgemässe | 7 | 7,20±0,30 | 6,60±0,32 |
| Verbindung II | I4 | 7,60±0,32 | 6,8I±0,30 |
| (I3,6 mg/kg) | 30 | 6,40±0,42 | 6,48±0,I5 |
| Erfindungsgemässe | 7 | 8,I0±0,23 | 6,4I±0,2I |
| Verbindung III | I4 | 8,40±0,42 | 6,68±0,29 |
| (I3,6 mg/kg) | 30 | 8,50±0,30 | 6,32±0,24 |
| DNS (6,0 mg/kg) | 7 | 9,42±0,37 | 7,92±0,3I |
| | I4 | I0,8±0,24 | 7,80±0,50 |
| | 30 | I0,0±0,I2 | 6,49±0,50 |

Die angeführten Ergebnisse zeigen, dass die DNS den Gehalt an Antikörpern gegenüber den Hammelerythrozyten im Blutserum erhöht, und das CDDP und die beanspruchten Stoffe senken, dabei ist die immunodepressive Einwirkung der angemeldeten Stoffe weniger als bei dem CDDP ausgeprägt.

Es wurden Untersuchungen des Einflusses der bean -

spruchten Stoffe im Vergleich zu den CDDP- und DNS-Präparaten auf die Bildung von antikörperbildenden Zellen in der Milz von zwei gegensätzlich auf die Hammelerythrozyte reagierenden Mäuse-Linien durchgeführt.

Den Mäusen der CBA-Linien und $C_{57}Bl_6$-Linie mit einem Gewicht von 20±2 g führte man intraperitoneal einmal das CDDP in einer Dosis von 8,I mg/kg, die DNS in einer Dosis von 6,0 mg/kg und die beanspruchten Stoffe in einer Dosis von I3,6 mg/kg ein.

In I Stunde wurden die Tiere immunisiert, indem man ihnen intraperitoneal $IO^8$ Zellen der Hammelerythrozyte einführte . Am 4. Tag ermittelte man die Anzahl der antikörperbildenden Zellen in der Milz der Tiere der Versuchs- und der Kontrollgruppe (ohne Einführung des Präparats). Der Gehalt an antikörperbildenden Zellen berechnete man auf $IO^6$ Splenozyte und auf die ganze Milz. Die Ergebnisse der Untersuchungen sind in der Tagelle 6 dargestellt.

Tabelle 6

| Einzuführende Stoffe | Gehalt an antikörperbildenden Zellen | | | |
|---|---|---|---|---|
| | Mäuse-Linien CBA | | Mäuse-Linie $C_{57}Bl_6$ | |
| | für $IO^6$ Zellen | für die ganze Milz x IOOO | für $IO^6$ Zellen | für die ganze Milz x IOOO |
| Kontrolle | I60,0±7,0 | 240,0±IO,2 | 72,0±4,0 | 9,4±I,3 |
| CDDP (8,I mg/kg-) | 42,0±3,8 | 63,0±2,2 | I3,3±I,24 | 2,2±0,96 |
| DNS (6,0 mg/kg) | 240,8±3,2 | 500,7± I7,4 | 8I,2±4,I | IO,3±I,I2 |
| Erfindungsgemässe Verbindung I (I3,6 mg/kg) | 5I,0±2,8 | 76,5±7,3 | 25,3±2,8 | 3,89±0,7 |
| Erfindungsgemässe Verbindung II (I3,6 mg/kg) | 52,7±4,I8 | 79,05±6,42 | 30,27±3,7 | 3,94±0,8 |
| Erfindungsgemässe Verbindung III (I3,6 mg/kg) | 54,8±5,82 | 82,20±7,24 | 32,72±4,02 | 4,26±0,8 |

Aus den in Tabelle 6 angeführten Angaben ist zu ersehen, dass die DNS den Gehalt an antikörperbildenden Zellen in der Milz bei der Mäusen erhöht, die sowohl wenig als auch stark auf die Hammelerythrozyte reagierenden Mäuse-Linien. Das CDDP und die beanspruchten Stoffe senken den Gehalt an antikörperbildenden Zellen in der Milz bei den Mäusen sowohl der CBA-Linie als auch der $C_{57}Bl_6$-Linie, die immunodepressive Wirkung der angemeldeten Stoffe ist jedoch weniger ausgeprägt als die des CDDP.

Somit erweisen sowohl die beanspruchten Stoffe als auch das CDDP eine ausgeprägte immunodepressive Wirkung auf das System der humoralen Immunität sowohl bei intakten Tieren als auch bei Tumorträgern. Sie vermindern die Bildung von Zellen, Antikörperproduzenten, wodurch die Anzahl von Antikörpern im Blutserum vermindert wird. Die immunodepressive Wirkung der beanspruchten Stoffe ist weniger als die des CDDP ausgeprägt.

Man untersuchte die Kennziffern der funktionellen Aktivität von Leber und Nieren bei Ratten. Den intakten Tieren führt man intraperitoneal dreimal in gleichen Portionen in einem Zeitabstand von einer Stunde wässerige Lösungen der erfindungsgemässen Verbindungen I und II und Zitrat-Salz-Lösungen von CDDP sowie der erfindungsgemässen Verbindung III in toxischen Zwischendosen gleich I/2 $LD_{50}$ ein, was insgesamt für das CDDP 9,0 mg/kg betrug, für die erfindungsgemässen Verbindungen I, II und III - 25,54 mg/kg, 33,0 mg/kg und 50,I mg/kg betrug. In 24 Stunden und in 7 Tagen tötete man die Tiere und ermittelte man einige biochemische Kenndaten des Blutes, die den funktionellen Zustand von Leber und Nieren charakterisieren. Die Ergebnisse der Versuche zeigten, dass am ersten Tag nach der Einführung des CDDP und der beanspruchten Stoffe in den Dosen gleich I/2 $LD_{50}$ eine statistisch zuverlässige Erhöhung der Transaminasen (Alaninaminotransferasen und Aspartataminotransferasen), den Billirubinpegel's sowie die Erhöhung der Menge von Harnstoff im Blutserum erfolgen. Am 7. Tag nach der Einführung der beanspruchten Stoffe normalisieren sich diese Kennziffern.

Bei den überlebten Tieren, die das CDDP bekamen, waren diese Kennziffern höher als normal. Die erzielten Ergebnisse zeigen, dass die angemeldeten Stoffe in den toxischen Zwischendosen gleich I/2 $LD_{50}$ eine umkehrbare nephrotoxische und hepatotoxische Wirkung aufweisen. Die Normalisierung der Kennziffern der funktionellen Aktivität der Leber und der Nieren bei Tieren, die das CDDP verabreichten, tritt am 7. Tag nicht ein.

Die Ergebnisse der durchgeführten biochemischen Untersuchungen werden durch histologische Untersuchungen von Leber und Nierengewebe bekräftigt.

Der im Ergebnis der durchgeführten Untersuchungen ermittelte Komplex von Eigenschaften: hohe Kennziffern des Hemmungsgrades des Wachstums eines Tumors, der Uberlebenskraft von Tieren und ihrer Lebensdauer bei der gleichzeitigen Senkung der Toxizität machen die angemeldeten Stoffe für die Entwicklung von effektiven Präparaten für die Behandlung bösartiger Tumore aussichtsvoll.

Ein zusätzlicher Vorteil der beanspruchten Stoffe besteht in dem Vorliegen ausgeprägter immunomodulierender Eigenschaften bei denselben infolge der Stimulierung der zellulären Immunität und einer mässigen Senkung der homoralen Immunität, was ebenfalls die Möglichkeit für die Erhöhung der Effektivität ihrer Anwendung bei der Chemotherapie bösartiger Tumore bewirkt.

Es wurden klinische Untersuchungen des erfindungsgemässen Arzneimittels antineoplastischer Wirkung durchgeführt, das als Wirkstoff die angemeldete Verbindung III enthält. Die klinischen Untersuchungen wurden an I02 Patienten mit Eierstocktumor und an 32 Patienten mit Lebertumor im IV. klinischen Stadium der Erkrankung im Alter von I4 bis 68 Jahren durchgeführt.

Bei dem Eierstocktumor im IV. klinischen Stadium wurde das erfindungsgemässe Präparat in Form einer Lösung (pH-Wert von 7,0 bis 7,5 und Ionenstärke - 0,02) in die Bauchhöhle am 3. und am 5. Tag nach einer Nichttotaloperation eingeführt. Das Präparat führte man dreimal zu je 380 mg in einem Volumen von 250 ml innerhalb von I bis

I,5 Stunden (bei Patienten mit einem Gewicht von 75 kg) mit Unterbrechungen von 2 bis 4 Tagen in Abhängigkeit von dem Zustand des jeweiligen Patienten ein. Die Gesamtdosis des erfindungsgemässen Präparates betrug von II50 bis I500 mg Wirkstoff für die gesamte Behandlungsdauer, was den 675 bis 850 mg reines CDDP äquivalent ist und auf das 4 bis 6fache die maximal zulässige Behandlungskursdosis von CDDP übertrifft, die gleich I20 bis I50 mg ist.

In drei Fällen führte man das erfindungsgemässe Präparat in den gleichen Dosen und unter den gleichen Bedingungen 4 bis 6 Wochen vor der Operation bei inoperablen Eierstocktumoren ein. Das verursachte die Lysis der Hauptmasse eines Tumors und das teilweise Zurückgehen von entfernten Metastasen, was es ermöglichte, später einen chirurgischen Eingriff vorzunehmen. Anschliessend wurde die Chemotherapie mit dem erfindungsgemässen Präparat fortgesetzt. Die Patienten mit dem Eierstocktumor der beschriebenen Gruppe erhielten I bis 4 Behandlungen mit Chemotherapie in einem Zeitabstand von 2 bis 4 Monaten ohne zwangsläufige Hydratation und eine verstärkte Diurese.

Bei dem primären und metastatischen Lebertumor im IV. klinischen Stadium wurde das erfindungsgemässe Präparat in Form einer Lösung trofpenweise innerhalb von 6 bis 8 Stunden in die Leberarterie oder in die Nabelvene eingeführt. In IO Fällen wurde das erfindungsgemässe Präparat in Form einer wässerigen Suspension selektiv unter Kontrolle der Echolokation zwei- oder dreimal in Dosen von 380 bis 760 mg in einem Volumen von IO bis 50 ml für einen Patienten mit einem Gewicht von 75 kg bis 80 kg eingeführt. Den Angaben der Echographie zufolge bleiben die Stoffe innerhalb von 4 bis 8 Monaten nach ihrer Einführung in einem Tumorherd erhalten.

In 4 bis 6 Wochen nach der durchgeführten Infusions- oder Lokalbehandlungen mit dem erfindungsgemässen Präparat verringerte sich die Grösse der Leber, sie wurde weich, schmerzlos das Schmerzsyndrom wurde praktisch verkürzt. Im weiteren erlaubte es, den chirurgischen Eingriff vorzunehmen, und zwar die Kryodestruktion.

Bei der Behandlung der Patienten mit dem Eierstocktumor und mit Lebertumor in IV. klinischen Stadium mit dem erfindungsgemässen Präparat wurde die Verbesserung des allgemeinen Zustandes, der Stimmungslage und des Appetits beobachtet.

Gleich nach der Einführung des angemeldeten Präparats oder nach Ablauf von 2 bis 3 Stunden wurde der Brechreiz oder das zwei- bis sechsmalige Erbrechen, manchmal eine kurzfristige Temperaturerhöhung auf 37,5°C nach der ersten Einführung beobachtet. Bei allen mit dem erfindungsgemässen Präparat behandelten Patienten wurden klinische Laboruntersuchungen von Urin und Blut durchgeführt. Es wurde festegestellt, dass der Eiweissgehalt im Urin sich unwesentlich gleich nach der Einführung des erfindungsgemässen Präparates erhöht und sich in I2 bis I8 Stunden normalisiert. Der Gehalt an Kreatin und Harnstoff im Blutserum der behandelten Patienten ging nicht über die Grenzwerte der physiologischen Schwankungen hinaus. Die erzielten Angaben beweisen das Fehlen nephrotoxischer Eigenschaften des erfindungsgemässen Präparates.

Die Veränderung des Gehaltes an Billirubin im Blutserum bei Behandlung des Eierstock- und Lebertumors gingen nicht über die Grenzwerte der physiologischen Schwankungen hinaus.

Bei Patienten mit dem Eierstocktumor (unter Fehlen von Metastasen in die Leber) übersteigt die Aktivität der Transaminasen vor Behandlung die Kennziffern der Norm auf das I,5fache. Bei der Behandlung mit dem erfindungsgemäss angemeldeten Präparat vergrössern sich diese Kennziffern noch mehr und übersteigen die Norm auf das 2fache. Die Normalisierung der Aktivität der Transaminasen wird gleich oder in einer Woche nach der Beendigung der ganzen Behandlung nachgewiesen.

Beim Lebertumor, sowohl primärem als auch metastatischem, übertrifft die Aktivität der Transaminasen die Kennziffern der Norm auf das 2fache. Bei der Behandlung mit dem erfindungsgemässen Präparat vergrössert sich die Aktivität der Transaminasen auf das 2,5 bis 3fache im

Vergleich zur Norm dabei überwiegt die Alaninaminotransferase über die Aspartatamihotransferase, was von dem Zellenzerfall des Lebertumors und von der hepatotoxischen Wirkung des Stoffes zeugt.

Die Normalisierung der Aktivität der Transaminasen wird in 2 bis 4 Wochen nach der Beendigung der Behandlung beobachtet. Hierdurch stimmten die Ergebnisse der klinischen Untersuchungen mit den Ergebnissen der Untersuchungen überein, die bei Versuchen an Tieren durchgeführt wurden und die auf eine unwesentliche nephorotoxische und hepatotoxische Wirkung des erfindungsgemässen Präparates sowie auf die Umkehrbarkeit der von ihm hervorgerufenen Veränderungen hindeutet.

Die klinischen Labroruntersuchungen der Kennziffern der zellulären und der humoralen Immunität und der Reserve des Knochenmarks nach der durchgeführten Chemotherapie mit dem erfindungsgemässen Präparat haben das Fehlen der hämotologischen Toxizität des Stoffes und seine immunomodulierende Wirkung nachgewiesen. Der Gehalt an T-Lymphozyten steigt nach der durchgeführten Chemotherapie bis zu einem Niveau an, das für gesunde Blutspender charakteristisch ist, und der Gehalt an B-Lymphozyten ist um das 2fache niedriger als in der Norm, der Gehalt an IgG etwas höher und an IgA und IgM niedriger als bei gesunden Blutspendern.

Die durchgeführten klinischen Untersuchungen haben ergeben, dass die Effektivität der Anwendung des erfindungsgemässen Präparates bei Patienten mit Eierstocktumor im IV. klinischen Stadium 90% erreicht, dabei wurden vollständigen Remissionen in 35% der Fälle, teilweise Remissionen und Stabilisierung des Prozesses in 55% der Fälle nachgeweisen, bei 10% der Patienten erwies sich die Anwendung des erfindungsgemässen Präparates als uneffektiv. Bei vollständiger klinischen Remission erreicht die Lebensdauer von Patienten 2,5 Jahre bei fortwährender Beobachtung.

Bei der Behandlung des Leberkarzinoms im IV. klinischen Stadium unter Verwendung des angemeldeten Präparates betrug die Lebensdauer der Patienten von 7,5 bis II

Monaten vom Beginn der Behandlung. Bei 3 Patienten übersteigt die Lebensdauer I8 Monate bei fortwährender Beobachtung.

Das erfindungsgemässe Präparat hat folgende Kontraindikationen: es kann nicht bei einer ausgeprägten Niereninsuffizienz, bei einer starken Entwässerung des Organismus und bei einem beträchtlichen Blutverlust angewendet werden.

Das erfindungsgemässe Präparat wird vorzugsweise in Form von Injektionslösungen beziehungsweise -suspensionen angewendet. Es wird für folgende Anwendung empfohlen:

I. Für Infusionstherapie: dreimal, intraperitoneal oder intrapleural zu je 380 mg in einem Volumen von 250 ml für einen Patienten mit einem Gewicht von 75 kg. Die Infusion wird innerhalb von I,0 bis I,5 Stunden mit einem Zeitabstand zwischen den Einführungen von 2 bis 3 Tagen in Abhängigkeit von dem Zustand des jeweiligen Patienten und mit Pausen zwischen den Behandlungen von 2 bis 4 Monaten vorgenommen.

2. Dreimal, langsam tropfenweise innerhalb von 6 bis 7 Stunden in die Leberarterie oder in die Nabelvene, zu je 380 mg in einem Volumen von 250 ml für einen Patienten mit einem Gewicht von 75 kg in einem Zeitabstand zwischen den Einführungen von 2 bis 3 Tagen in Abhängigkeit vom Zustand des jeweiligen Patienten und mit Pausen zwischen den Behandlungen von 2 bis 2,5 Monaten.

3. Lokal in den Tumor zu je 380 bis 760 mg in einem Volumen von IO bis 50 ml in Suspension dreimal für jeden Patienten mit einem Gewicht von 75 kg mit Pausen zwischen den Injektionen von 2 bis 3 Tagen in Abhängigkeit von seinem Zustand. Die Fortsetzung der Chemotherapie erfolgt im Tropfenverfahren, wie bereits oben erwähnt.

Die beanspruchten Verbindungen werden wie folgt hergestellt.

Desoxyribonukleinsäure mit einer Molekularmasse nicht unter $8 \cdot 10^6$ D wird mit cis-Dichloroamminplatin(II) oder mit teilweise hydrolysiertem cis-Dichlordiamminplatin(II) in einem wässerigen Medium beziehungsweise in einer Pufferlösung mit einem pH-Wert von 7,0 bis 7,5 und einer Ionenstärke von 0,0I bis 0,02 bii einer Temperatur nicht unter dem Schmelzpunkt der Desoxyribonukleinsäure mit anschliessender Isolierung des Endproduktes umgesetzt.

Die beanspruchte Verbindung I Poly{hexakis [chloroammindiaquaplatin(II)]} - μ - desoxyribonukleat und die beanspruchte Verbindung (III) Poly{hexakis [chloroamminaquaplatin(II)]} -μ- desoxyribonukleat gewinnt man durch Umsetzung der nativen Desoxyribonukleinsäure mit cis-Dichlorodiamminplatin und die angemeldete Verbindung II Poly{bis [Hydroxochloroamminaquaplatin(II)] tetrakis [chloroammindiaquaplatin(II)]} -μ- desoxyribonukleat gewinnt man durch Umsetzung der Desoxyribonukleinsäure mit teilweise hydrolysiertem cis-Dichlorodiamminplatin.

Den Prozess führt man in einem wässerigen Medium mit einem pH-Wert von 7,0 bis 7,5 und einer Ionenstärke von 0,0I bis 0,02 bei einer Temperatur nicht unter dem Schmelzpunkt der DNS bis zur Beendigung der Reaktion durch. Das Abklingen der Reaktion prüft man spektrophotometrisch nach der Erreichung der Parameter der UV-Spektren.

Das Endprodukt isoliert man aus dem Reaktionsgemisch, indem man das Ammoniumchlorid und den Uberschuss an Wasser mit lyophiler Trocknung entfernt. Das Endprodukt kann auch in Form einer Lösung ausgeschieden werden. Chemische Zusammensetzung der angemeldeten Stoffe in einer Lösung und im getrockneten Zustand ist identisch.

Zur besseren Erläuterung der vorliegenden Erfindung werden nachstehende Beispiele für die Herstellung der beanspruchten Verbindungen angeführt.

Beispiel I

Einer Lösung von 500 mg der nativen DNS mit einer Molekularmasse von $8{,}5 \cdot 10^{6}$ D in 375 ml Wasser, die bis auf den Schmelzpunkt der DNS (78,0°C) erwärmt wird, setzt man eine Lösung von 675 mg cis-Dichlorodiamminplatin(II) in 375 ml Wasser zu, die auf die gleiche Temperatur erwärmt wird. Das Reagenziengemisch lässt man innerhalb von I5 Minuten unter Vermischen und einer Temperatur von 78,0°C

stehen, dann kühlt man es auf Raumtemperatur ab und setzt man der lyophilen Trocknung aus.

Man erhält II05 (90%) Poly{hexakis-[chloroammin-diaquaplatin(II)]}-$\mu$-desoxyribonukleat in Form eines feinkristallinen gelben Pulvers, das in Wasser, schwerlöslich ist mit folgenden Parametern des UV-Spektrums einer 0,002%igen Lösung in Wasser $\lambda_{max}$ = 268,8 = I00080 $l \cdot Mol^{-1} \cdot cm^{-I}$ und mit einer Molekularmasse von $6,2 \cdot IO^6$ D.
Gefunden, %: Pt 38,46; Cl 7,38; C I5,6I; N 9,36; H 3,09; P 4,67.
$C_{39}H_{9I}O_{44}N_{2I}Cl_6P_4Pt_6$.
Berechnet, %: Pt 38,II; C I6,94; C I5,25; N 9,58; H 2,97; P 4,I7.

Beispiel 2

Die Prozessführung erfolgt analog der in Beispiel I beschriebenen mit Ausnahme dessen, dass das Endprodukt aus dem Reaktionsgemisch nicht ausgeschieden wird. Der UV-Spektrum des gewonnenen Stoffes in einer Lösung wird charakterisiert durch $\lambda_{max}$= 268,8 nm und $E_{268,8}$ = I0000 $l \cdot Mol^{-I} \cdot cm^{-I}$. Seine Molekularmasse in einer Lösung ist gleich $6,0 \cdot IO^{-6}$ D. Der gewonnene Stoff ist in seiner chemischen Zusammensetzung dem in Beispiel I gewonnenen Stoff identisch.

Beispiel 3

Die Prozessführung erfolgt analog der in Beispiel I beschriebenen mit Ausnahme dessen, dass man als native Desoxyribonukleinsäure eine native DNS mit einer Molekularmasse von $I0,6 \cdot IO^6$ D verwendet. Man erhält ein Produkt, dessen UV-Spektrum in einer Lösung durch folgende Parameter charakterisiert wird $\lambda_{max}$ = 268,7 nm, $E_{268,7}$ = I0080, und die Molekularmasse ist gleich $6,2 \cdot IO^6$ D. Der gewonnene Stoff ist in seiner chemischen Zusammensetzung dem in Beispiel I gewonnenen Stoff identisch.

Beispiel 4

Eine Lösung von 405 mg cis-Dichlorodiamminplatin(II) in 250 ml Wasser, die bei Raumtemperatur hergestellt wird, erwärmt man bis auf den Schmelzpunkt der nativen DNS

(78,0°C) innerhalb von einer Stunde, setzt man diese einer Lösung von 333 mg der nativen DNS mit einer Molekularmasse von $II,2 \cdot IO^6$ in 250 ml Wasser zu, die auch auf 78,0°C erwärmt wird, und man lässt das Gemisch unter Vermischen bei der gleichen Temperatur innerhalb von I5 Minuten stehen. Das Gemisch kühlt man bei Raumtemperatur ab und setzt man der lyophilen Trocknung aus.

Man erhält 730 mg (93%) Poly{bis [hydroxochloroamminaquaplatin(II)] tetrakis [chloroammindiaquaplatin(II)]} -$\mu$- disoxyribonukleat in Form eines feinkristallinen gelben Pulvers, das in Wasser schwerlöslich ist, mit folgenden Parametern des UV-Spektrums einer 0,0075%igen Lösung in Wasser $\lambda_{max}$ = 265,7 nm, $E_{265,7}$ = 9980 $l \cdot Mol^{-I} cm^{-I}$ mit einer Molekularmasse von $6,0 \cdot IO^6$ D und einer intensiven Absorptionsbande im IR-Spektrum bei 3295 $cm^{-I}$, die der Valenzschwankung der OH-Gruppe in der PtOH-Gruppierung entspricht.

Gefunden, %: Pt 39,97; Cl 7,38; C I5,53; N 9,38; H 2,98; P 4,07.

$C_{39}H_{9I}O_{44}N_{2I}Cl_6P_4Pt_6$.

Berechnet, %: Pt 38,II; Cl 6,94; C I5,25; N 9,58; H 2,97; P 4,I7.

Beispiel 5

Die Prozessführung erfolgt analog der in Beispiel 4 beschreebenen mit Ausnahme dessen, dass das Endprodukt aus der Lösung nicht ausgeschieden wird. Der UV-Spektrum des gewonnenen Stoffes in einer Lösung wird durch folgende Parameter charakterisiert $\lambda_{max}$ = 265,8 nm, $E_{265,8}$ = I0050 $l \cdot Mol^{-I} cm^{-I}$. Die Molekularmasse des gewonnenen Stoffes in der Lösung beträgt $6,2 \cdot IO^6$ D. Der gewonnene Stoff ist in seiner chemischen Zusammensetzung dem in Beispiel 4 gewonnenen identisch.

Beispiel 6

Die Prozessführung erfolgt analog der in Beispiel 4 beschriebenen mit Ausnahme dessen, dass man als native DNS eine native DNS mit einer Molekularmasse von $9,6 \cdot IO^6$ D. verwendet. Man erhält ein Produkt mit einer Molekularmas-

se von 5,9·I0$^{6}$ D, dessen UV-Spektrum durch folgende Parameter charakterisiert wird $\lambda_{max}$ = 265,7 nm, $E_{265,7}$ = 9990·l·$Mol^{-I}cm^{-I}$. Der gewonnene Stoff ist in seiner chemischen Zusammensetzung dem in Beispiel 4 gewonnenen identisch.

Beispiel 7

Einer Zitrat-Salz-lösung (I5 mMol/l Natriumchlorid + I,5 mMol/l Natriumzitrat in Wasser), die 500 mg der nativen DNS mit einer Molekularmasse von I0,6·I0$^{6}$ D in 375 ml Lösung enthält, die bis auf den Schmelzpunkt der nativen DNS (78,0°C) erwärmt wird, setzt man 675 mg cis-Dichlorodiamminplatin(II) in 375 ml einer analogen Zitrat-Salz-Lösung zu, die auf die gleiche Temperatur erwärmt wird. Das Reagenziengemisch lässt man innerhalb von I5 Minuten bei der gleichen Temperatur und unter Vermischen stehen, dann kühlt man es auf Raumtemperatur ab und setzt man der lyophilen Trocknung aus. Man erhält I,8 g Lyophilisat, das ein Gemisch aus der beanspruchten Verbindung III, dem Natriumchlorid und Natriumzitrat darstellt. Den 250 mg Lyophilisat setzt man 50 ml destilliertes Wasser zu und vermischt man sorgfältig. Die angefallene Suspension zentrifugiert man bei Raumtemperatur mit einer Geschwindigkeit von 7000 U/min innerhalb von I5 Minuten. Der Niederschlag wird aus der Lösung dekantiert, mit Wasser, Äthylalkohol und Äthyläther gewaschen und an der Luft getrocknet. Man erhält 90 mg (60%) der Theorie) reines Poly {hexakis [chloroamminaquaplatin(II)]} -μ- desoxyribonukleat in Form eines feinkristallinen dunkelgelben Pulvers, begrenzt löslich in Wasser mit folgenden Parametern des UV-Spektrums einer 0,05%igen Lösung in Wasser, die I8 mMol/l Natriumchlorid und I,8 mMol/l Natriumzitrat enthält, $\lambda_{max}$ = 266,2 nm, $E_{266,2}$ = I0000 l·$Mol^{-I}cm^{-I}$ mit einer Molekularmasse von 6,2·I0$^{6}$ D.

Gefunden,%: Pt 39,8I; Cl 7,40; C I5,62; N I0,II; H 2,88; P 4,33.

$C_{39}H_{79}O_{38}N_{2I}Cl_{6}P_{4}Pt_{6}$.

Berechnet, %: Pt 39,58; Cl 7,2I; C I5,88; N 9,95; H 2,67; P 4,I9.

Beispiel 8

Die Prozessführung erfolgt analog der in Beispiel 7 beschriebenen mit Ausnahme dessen, dass das Endprodukt aus dem Reaktionsgemisch nicht ausgeschieden wird. Der UV-Spektrum des gewonnenen Stoffes in einer Lösung wird durch folgende Parameter charakterisiert $\lambda_{max}$ = 266,2 nm, $E_{266,2}$ = IOIOO $l \cdot Mol^{-I} \cdot cm^{-I}$. Seine Molekularmasse beträgt $5,9 \cdot IO^6$ D. Der gewonnene Stoff ist in seiner chemischen Zusammensetzung dem in Beispiel 7 gewonnenen identisch.

Beispiel 9

Die Prozessführung erfolgt analog der in Beispiel 7 beschriebenen mit Ausnahme dessen, dass man als native DNS eine native DNS mit einer Molekularmasse von $9,6 \cdot IO^6$ D verwendet.

Man erhält ein Produkt mit einer Molekularmasse von $6,0 \cdot IO^6$ D und einem UV-Spektrum, der durch $\lambda_{max}$ = 266,2 nm, $E_{266,2}$ = IOO2O $l \cdot Mol^{-I} \cdot cm^{-I}$ charakterisiert wird. Der gewonnene Stoff ist in seiner chemischen Zusammensetzung dem in Beispiel 7 gewonnenen identisch.

Gewerbliche Anwendbarkeit

Die erfindungsgemässen neuen Verbindungen, Derivate des Platin(II) mit einem Polyanion der Desoxyribonukleinsäure besitzen eine antineoplatische Wirkung und finden Anwendung in der Medizin als Wirkstoffe eines Arzneimittels für Chemotherapie von Eierstock-, Lungen-, Leber- und Gallenblasekarzinom.

PATENTANSPRUCHE:

I. Derivate des Platin(II) mit einem Polyanion der Desoxyribonukleinsäure allgemeiner Formel: $\{[PtCl(NH_3)]_6\ x\ ^{-m}(OH)_x(H_2O)_y\}_n$-DNS $^{-m}$, worin DNS$^{-m}$ = $(C_{39}H_{55-r}O_{32}N_{I5}P_4)_n^{-m}$
n = 2000±200;
bei x = 0, y = I2, m = 6n, r = 6;
bei x = 2, y = I0, m = 4n, r = 4;
bei x = 0, y = 6, m = 6n, r = 6.

2. Verfahren zur Herstellung von Derivaten des Platin(II) mit einem Polyanion der Desoxyribonukleinsäure nach Anspruch I, dadurch gekennzeichnet, dass man die Umsetzung der Desoxyribonukleinsäure mit einer Molekularmasse nicht unter 8 Millionen Dalton mit cis-Dichlorodiamminplatin(II) oder mit teilweise hydrolysiertem cis-Dichlorodiamminplatin(II) in einem wässerigen Medium beziehungsweise in einer Pufferlösung mit einem pH-Wert von 7,0 bis 7,5 und einer Ionenstärke von 0,0I bis 0,02 bei einer Temperatur nicht unter dem Schmelzpunkt der Desoxyribonukleinsäure mit anschliessender Isolierung des Endproduktes durchführt.

3. Arzneimittel mit antineoplastischer Wirkung, das einen Wirkstoff und ein pharmazeutisches Verdünnungsmittel vorsieht, dadurch gekennzeichnet, dass es als Wirkstoff Poly{hexakis[chloroamminaquaplatin(II)]} - $\mu$ - desoxyribonukleat folgender Formel aufweist: $\{[PtCl(NH_3)]_6\ (H_2O)_6\}_n$-DNS$^{-m}$, worin DNS$^{-m}$ = $(C_{39}H_{49}O_{32}N_{I5}P_4)_n^{-m}$, n = 2I00±I00, m = 6n, nach Anspruch I.

5. Arzneimittel nach Anspruch 3, in Form von Injektionslösungen, dadurch gekennzeichnet, dass es den Wirkstoff in einer Menge von 0,I5 Masse% enthält.

5. Arzneimittel nach Anspruch 3 in Form von Injektionslösungen, dadurch gekennzeichnet, dass es als pharmazeutisches Verdünnungsmittel eine wässerige Lösung aus 0,085 Masse% Natriumchlorid und 0,053 Masse% Natriumzitrat enthält.

6. Arzneimittel nach Anspruch 3 in Form von Injektionssuspensionen, dadurch gekennzeichnet, dass es den Wirkstoff in einer Menge von I,5 bis 3,7 Masse% enthält.

7. Arzneimittel nach Anspruch 3, in Form von Injektionssuspensionen, dadurch gekennzeichnet, dass es als pharmazeutisches Verdünnungsmittel eine wässerige Lösung aus 0,85 bis 2,I Masse% Natriumchlorid und von 0,53 bis I,3 Masse% Natriumzitrat enthält.

89907570.1 00-70

# INTERNATIONAL SEARCH REPORT

International Application No PCT/SU 89/00084

**I. CLASSIFICATION OF SUBJECT MATTER** (if several classification symbols apply, indicate all) [6]

According to International Patent Classification (IPC) or to both National Classification and IPC

Int.Cl.4 A61K 37/10 , 33/24 , 9/08 , 31/28

**II. FIELDS SEARCHED**

Minimum Documentation Searched [7]

| Classification System | Classification Symbols |
|---|---|
| Int.Cl.4 | A61K 9/08,31/28, A61K 33/24,37/10, C07F 15/00 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | WO,AI, 85/03296 (INSTITUE OBSCHEI I NEORGANICHESKOI ICHIMII IMENI N.S. KURNAKOVA AKADEMII NAUK SSSR et al) 1 August 1985 (01.08.85)see the claims & GB,2163163 | 1 |
| A | US,A, 4544759 (AMERICAN CYANAMID COMPANY) 1 October 1985 (01.10.85) the claims | 1-2 |
| A | US,A, 4562275 (BRISTOL-MYERS CO) 31 December 1985 (31.12.85) the claims & EP,155705 | 1-2 |
| A | EP,AI,0210291 (AMERICAN CYANAMID COMPANY) 4 February 1987 (04.02.87) columns 20-21 | 3-7 |
| A | US, 4504487 (CHIKAO YOSHIKUMI et al) 12 March 1985 (12.03.85) the claims & EP,B1, 41792 | 3-7 |
| A | GB,B, 1440626 (COCIETA FARMACEUTI ITALIA) 23 June 1976 (23.06.76)page 1, columns 33-45 | 3 |

.../...

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 29 June 1989 (29.06.89) | 13 September 1989 (13.09.89) |
| **International Searching Authority** | **Signature of Authorized Officer** |
| ISA/SU | |

International Application No. PCT/SU 89/00084

**III. DOCUMENTS CONSIDERED TO BE RELEVANT (CONTINUED FROM THE SECOND SHEET)**

| Category * | Citation of Document, with indication, where appropriate, of the relevant passages | Relevant to Claim No |
|---|---|---|
| A | US,A, 4758588 (DAVID B,BROWN et al)<br>19 July 1988 (19.07.88)<br>column 24, pages 40-63<br>& EP,107-104; JP,128-388<br>--- | 3-7 |
| A | EP,A2,0280474 (JOHNSON MATTHEJ,ING)<br>31 August 1988 (31.08.88) the claims | 3-7 |